# EUROPÄISCHE PATENTANMELDUNG

(11) **EP 4 296 261 A1**
(43) Veröffentlichungstag der Anmeldung: **27.12.2023**
(21) Anmeldenummer: 22180310.9
(22) Anmeldetag: 22.06.2022
(51) Int. Cl.: C07C 263/10, C07C 265/14, C07D 307/00, B01J 3/00

(54) **VERFAHREN UND VORRICHTUNG ZUR HERSTELLUNG VON ISOCYANATEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von Isocyanaten durch Gasphasenphosgenierung der korrespondierenden Amine in einer Phosgenierungsanlage, bei dem insbesondere Probleme infolge der Bildung von Ablagerungen in Apparaten der Phosgenierungsanlage, wie insbesondere der Reaktionsstrecke, während der Inbetriebnahme (dem Anfahren) des Verfahrens möglichst vermieden werden. Darüber hinaus betrifft die Erfindung eine Vorrichtung zur Durchführung des erfindungsgemäßen Verfahrens sowie ein Verfahren zum Anfahren einer Phosgenierungsanlage.

## Beschreibung

Die Erfindung betrifft ein Verfahren zur Herstellung von Isocyanaten durch Gasphasenphosgenierung der korrespondierenden Amine in einer Phosgenierungsanlage, bei dem insbesondere Probleme infolge der Bildung von Ablagerungen in Apparaten der Phosgenierungsanlage, wie insbesondere der Reaktionsstrecke, während der Inbetriebnahme (dem Anfahren) des Verfahrens möglichst vermieden werden. Darüber hinaus betrifft die Erfindung eine Vorrichtung zur Durchführung des erfindungsgemäßen Verfahrens sowie ein Verfahren zum Anfahren einer Phosgenierungsanlage.

Die großtechnische Herstellung von Isocyanaten durch Umsetzung der korrespondierenden Amine mit Phosgen ist seit längerem aus dem Stand der Technik bekannt, wobei die Reaktion in der Gas- oder Flüssigphase kontinuierlich oder diskontinuierlich stattfinden kann. Von besonderer Bedeutung sind Di- oder Polyisocyanate, die sich zur Herstellung von Polymeren, wie beispielswiese Polyurethanen, Polythiourethanen, Polyisocyanuraten oder Polyharnstoffen eignen. Es kommen dabei sowohl aromatische Isocyanate wie beispielsweise Methylendiphenyldiisocyanat bzw. dessen höhere Homologe, Toluylendiisocyanat als auch aliphatische Isocyanate wie beispielsweise Hexamethylendiisocyanat, Pentamethylendiisocyanat oder Isophorondiisocyanat zum Einsatz.

Die moderne großtechnische Herstellung von Polyisocyanaten erfolgt kontinuierlich. Dabei kommt es immer wieder zu geplanten Abschaltungen oder auch ungeplanten Ausfällen des Prozesses. Die Gründe hierfür sind vielfältig und reichen vom Ausfall von Apparaten oder Steuerungseinrichungen über Störungen in der Rohstoffversorgung bis hin zu wirtschaftlichen Gründen wie einer zu schwachen Nachfrage nach dem Produkt.

Die WO2015/144658 A1 befasst sich ausdrücklich mit solchen Abweichungen vom Normalbetrieb und gibt Hinweise darauf, wie bei solchen Abweichungen das Risiko von Ablagerungen in der Mischzone zur Vermischung von Amin und Phosgen beziehungsweise in der strömungstechnisch danach angeordneten Reaktionszone minimiert werden kann. Zu diesem Zweck wird empfohlen, stets einen Überschuss an Phosgen gegenüber dem Amin sicherzustellen, indem beispielsweise beim Anfahren der kontinuierlichen Produktion der Reaktor zunächst nur mit Lösungsmittel und Phosgen aufgeheizt und erst dann die Aminzufuhr gestartet wird, beziehungsweise beim Abfahren der kontinuierlichen Produktion zunächst die Aminzufuhr und erst dann die Phosgenzufuhr unterbrochen wird.

Auch in den der Mischzone und der Reaktionszone vorgelagerten Apparaten, die der Vorbereitung des Amins, also beispielsweise dessen Temperierung, Verdampfung oder Überhitzung, dienen, gibt es jedoch bei einem Anlagenstillstand Probleme, auf die in den genannten Dokumenten nicht eingegangen wird. So gibt es beispielsweise eine Reihe von Aminen, die bei Raumtemperatur erstarren, so dass einerseits die Wiederinbetriebnahme nach einem Stillstand erheblich erschwert ist und andererseits die Reinigung von Apparaten, die solche Amine enthalten, z.B. für Wartungszwecke, deutlich erschwert ist.

Speziell mit der Inbetriebnahme einer Gasphasenphosgenierungsanlage befasst sich die WO2015/144681 A1. Hier wird offenbart, dass die Inbetriebnahme einer solchen Anlage ein kritischer Verfahrensschritt ist. Es wird beschrieben, dass es dabei zur Bildung von Amin-Tröpfchen sowie von Feststoffen wie Polyharnstoffen oder Aminhydrochloriden und in der Folge einer Erhöhung des zur ausreichenden Durchströmung der Anlage erforderlichen Differenzdrucks kommen kann. Zur Minimierung dieser Probleme wird vor allem empfohlen, auch während des Inbetriebnahmevorgangs die Eduktströme so aufeinander abzustimmen, dass stets ein stöchiometrischer Überschuss an Phosgen in Bezug auf die primären Aminogruppen des Amins vorliegt.

Darüber hinaus hat sich in der betrieblichen Praxis gerade für die Phosgenierung von Aminen in der Gasphase gezeigt, dass es Amine gibt, die unter weniger optimalen Phosgenierbedingungen, wie sie beim Anfahren des Prozesses herrschen, häufiger Probleme bereiten, also beispielsweise zur raschen Bildung von Ablagerungen neigen und solche, die sich auch unter diesen Prozessbedingungen eher gutmütig verhalten, wie insbesondere 1-Amino-3,5,5-trimethyl-5-aminomethylcyclohexan (IPDA). Auch bei konsequenter Anwendung der Lehren aus WO2015/144681 A1 und WO2015/144658 A1 lässt sich nicht verhindern, dass es mitunter eine Stunde oder mehr dauern kann, bis sich die im kontinuierlichen Betrieb vorliegenden Temperaturprofile in der Reaktionszone eingestellt haben. Dies liegt daran (ohne an eine Theorie gebunden zu sein), dass der Phosgenierungsreaktor zwar zuvor aufgeheizt wird, es aufgrund der exothermen Reaktion aber zu einer weiteren Erwärmung kommt. In der Anfangsphase sind Teile der Reaktionszone folglich noch nicht bei optimaler Betriebstemperatur, was bei der Phosgenierung von eher problematischen Aminen zu einer frühen und nicht reversiblen Bildung von Ablagerungen führen kann. Diese Ablagerungen wiederum können zu einem deutlichen Druckanstieg, beispielsweise in der Amin-Zuleitung der Amin-Vorrichtung, führen. Dies lässt sich auch nicht durch ein stärkeres Vorheizen des Reaktors lösen. Erstens ist dies technisch schwierig umzusetzen und zweitens führt dann die einsetzende exotherme Reaktion zu einer Übertemperatur im Reaktionsgemisch und damit zu einer Schädigung des Produkts.

Die Phosgenierung von Mischungen verschiedener Amine, wobei mindestens ein Amin eine aliphatisch gebundene primäre Aminogruppe enthält, ist in DE2249459 A1 offenbart. Dort werden Mischungen von Aminen phosgeniert und anschließend eines der Isocyanate aus dem Reaktionsgemisch abgetrennt. Es wird auf den wirtschaftlichen Vorteil hingewiesen, der sich bei einer solchen Vorgehensweise aus der besseren Anlagenauslastung gerade für speziellere Isocyanate ergibt. In allen beispielhaft offenbarten Reaktionen wird zunächst eine Mischung aus zwei oder mehr Aminen in einem festen Verhältnis hergestellt und diese Mischung dann in die Phosgeniervorrichtung eingebracht. Für ein kontinuierliches Verfahren wird es bevorzugt, die Amine als Gemisch oder zumindest gleichzeitig dem Phosgen zuzusetzen. Werden die Amine als Gemisch vorgelegt, ist einerseits das Mischungsverhältnis festgelegt und andererseits können die Probleme, die mit anspruchsvolleren Aminen einhergehen nicht sicher vermieden werden.

Es besteht daher ein Bedarf nach einem Verfahren und einer Vorrichtung zur Herstellung von Isocyanaten, die die genannten Probleme umgehen und die einen verbesserten Phosgenierprozesses erlauben, insbesondere während der Inbetriebnahme der Phosgenierungsanlage, eine Phosgenierung von Aminen in variablen Mischungsverhältnissen ermöglichen und die Reinigung von dem Phosgenierungsreaktor vorgelagerten Apparaten im Amin-Weg vereinfachen.

Die Aufgabe der vorliegenden Erfindung bestand demnach darin, ein Verfahren zur Herstellung von Isocyanaten durch Gasphasenphosgenierung der korrespondierenden Amine sowie eine Phosgenierungsanlage zur Durchführung dieses Verfahrens bereitzustellen, wodurch insbesondere die vorgenannten Probleme beseitigt oder zumindest verringert werden. Im Besonderen sollte durch das Verfahren und die Vorrichtung ein Druckanstieg in den Apparaten der Phosgenierungsanlage, wie der Aminstrom-Zuleitung der Aminstrom-Vorrichtung, vor allem bei der Inbetriebnahme (dem Anfahren) der Phosgenierungsanlage vermieden oder zumindest deutlich reduziert werden, der bei der Herstellung von Isocyanaten aus den zu einem solchen Druckanstieg führenden korrespondierenden Aminen in Anlagen und Verfahren des Standes der Technik für gewöhnlich auftritt. Vorzugsweise sollte ein solcher Druckanstieg auch während der Produktionsphase vermieden oder möglichst lange hinausgezögert werden.

Diesem Bedarf Rechnung tragend ist ein Gegenstand der vorliegenden Erfindung ein Verfahren zur Herstellung von Isocyanaten durch Gasphasenphosgenierung der korrespondierenden Amine in einer Phosgenierungsanlage, welche
(a) mindestens eine Phosgenstrom-Vorrichtung zur Bereitstellung eines gasförmigen Phosgenstroms, gegebenenfalls umfassend neben Phosgen einen Inertstoff im Phosgenstrom
(b) gegebenenfalls eine Inertstoffstrom-Vorrichtung zur Bereitstellung eines Inertstoffstroms,
(c) mindestens eine Aminstrom-Vorrichtung zur Bereitstellung eines gasförmigen Aminstroms, gegebenenfalls umfassend neben Aminen einen Inertstoff im Aminstrom;
(d) einen Phosgenierungsreaktor zur Vermischung der Ströme aus (a) bis (c) und Umsetzung des Aminstroms mit dem Phosgenstrom,
umfasst oder daraus besteht, wobei zur Inbetriebnahme der Phosgenierungsanlage die folgenden Schritte durchlaufen werden:
(A) Beschickung des Phosgenierungsreaktors mit dem gasförmigen Phosgenstrom und gegebenenfalls dem Inertstoffstrom sowie anschließend kontinuierliche Zufuhr des gasförmigen Phosgenstroms und gegebenenfalls des Inertstoffstroms in den Phosgenierungsreaktor,
(B) Kontinuierliche Zufuhr des Aminstroms in den Phosgenierungsreaktor,
dadurch gekennzeichnet, dass der Schritt (B) eine Anlaufphase, eine sich hieran anschließende Reduktionsphase und eine der Reduktionsphase nachgelagerte Produktionsphase umfasst, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird und wobei
- das erste Amin ausgewählt ist aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon; und
- das zweite Amin ausgewählt ist aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, 1-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon.

Ferner betrifft die Erfindung eine Phosgenierungsanlage zur Durchführung des erfindungsgemäßen Verfahrens, umfassend oder bestehend aus
(a) mindestens eine Phosgenstrom-Vorrichtung zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung zur Bereitstellung eines gasförmigen Aminstroms, gegebenenfalls umfassend neben Aminen einen Inertstoff im Aminstrom; und
(d) einen Phosgenierungsreaktor zur Vermischung der Ströme aus (a) bis (c) und Umsetzung des Aminstroms mit dem Phosgenstrom;
dadurch gekennzeichnet, dass die Aminstrom-Vorrichtung ausgestaltet ist, den Aminstrom in der Weise bereitzustellen, dass in einer Anlaufphase der Aminstrom mindestens ein erstes Amin sowie optional ein zweites Amin umfasst und der Massenanteil des ersten Amins an der Gesamtheit der Amine im Aminstrom, welcher dem Phosgenierungsreaktor zugeführt werden kann, mindestens 90,0 Gew.-%, bevorzugt 95,0 bis 100 Gew.-%, weiter bevorzugt 97,5 bis 100 Gew.-%, besonders bevorzugt 99,0 bis 100,0 Gew.-%, ganz besonders bevorzugt 100 Gew.-% ausmacht, dieser Massenanteil dann während einer auf die Anlaufphase folgenden Reduktionsphase auf 85,0 Gew.-% oder weniger, bevorzugt 0 bis 80,0 Gew.-%, weiter bevorzugt 0,1 bis 50,0 Gew.-%, besonders bevorzugt auf 1,0 bis 40,0 Gew.-%, ganz besonders bevorzugt auf 5,0 bis 30,0 Gew.-% reduziert und dieser Massenanteil dann in einer sich an die Reduktionsphase anschließenden Produktionsphase aufrechterhalten werden kann, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-%, bevorzugt maximal 0,2 Gew.-% von weiteren organischen Aminen, welche von dem ersten und zweiten Amin verschieden sind, gebildet ist.

Außerdem ist die Erfindung auf ein Verfahren zum Anfahren einer Phosgenierungsanalage gerichtet, welche
(a) mindestens eine Phosgenstrom-Vorrichtung zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung zur Bereitstellung eines gasförmigen Aminstroms, gegebenenfalls umfassend neben Aminen einen Inertstoff im Aminstrom; und
(d) einen Phosgenierungsreaktor zur Vermischung der Ströme aus (a) bis (c) und Umsetzung des Aminstroms mit dem Phosgenstrom;
umfasst oder daraus besteht, wobei zur Inbetriebnahme der Phosgenierungsanlage die folgenden Schritte durchlaufen werden:
(A) Beschickung des Phosgenierungsreaktors mit dem gasförmigen Phosgenstrom und gegebenenfalls des Inertstoffstroms sowie anschließend kontinuierliche Zufuhr des gasförmigen Phosgenstroms und gegebenenfalls des Inertstoffstroms in den Phosgenierungsreaktor,
(B) Kontinuierliche Zufuhr des Aminstroms in den Phosgenierungsreaktor,
dadurch gekennzeichnet, dass der Schritt (B) eine Anlaufphase, eine sich hieran anschließende Reduktionsphase und eine der Reduktionsphase nachgelagerte Produktionsphase umfasst, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird und
- das erste Amin ausgewählt ist aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon; und
- das zweite Amin ausgewählt ist aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, l-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon.

Das Wort "*ein*" ist im Rahmen dieser Erfindung im Zusammenhang mit zählbaren Größen lediglich als unbestimmter Artikel und nur dann als Zahlwort zu verstehen, wenn dies ausdrücklich, etwa durch den Zusatz "*genau* ein" gesagt wird. Ausdrücke wie "ein Wärmetauscher" oder "ein Aminstrom" schließen also die Möglichkeit des Vorhandenseins weiterer Wärmetauscher oder Aminströme nicht aus.

Der Begriff "gasförmig" ist so zu verstehen, dass dadurch auch ein Tröpfchenmitriss umfasst wird.

### Phosgenierungsanlage:

Die Phosgenierungsanlage umfasst oder besteht aus
(a) mindestens eine Phosgenstrom-Vorrichtung zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung zur Bereitstellung eines gasförmigen Aminstroms, gegebenenfalls umfassend neben Aminen einen Inertstoff im Aminstrom; und
(d) einen Phosgenierungsreaktor zur Vermischung der Ströme aus (a) bis (c) und Umsetzung des Aminstroms mit dem Phosgenstrom.

Die Phosgenstrom-Vorrichtung, die optionale Inertstoffstrom-Vorrichtung, die Aminstrom-Vorrichtung sowie der Phosgenierungsreaktor können unabhängig voneinander weitere Apparate umfassen, die für den Betrieb der Phosgnierungsanlage dienlich sind, wie beispielsweise Vorlagebehälter für die Ausgangsstoffe, Wärmetauscher zur Temperierung oder gegebenenfalls Verdampfung von Ausgangsstoffen, Einrichtungen zur Aufbereitung der Reaktionsmischung nach erfolgter Umsetzung von Amin mit Phosgen.

Vorzugsweise umfasst die Phosgenstrom-Vorrichtung mindestens einen Wärmetauscher zur Erwärmung des Phosgenstroms und/oder des Inertstoffstroms.

Die Aminstrom-Vorrichtung ist ausgestaltet den Aminstrom in der Weise bereitzustellen, dass in einer Anlaufphase der Aminstrom mindestens ein erstes Amin sowie optional ein zweites Amin umfasst und der Massenanteil des ersten Amins an der Gesamtheit der Amine im Aminstrom, welcher dem Phosgenierungsreaktor zugeführt werden kann, mindestens 90,0 Gew.-%, bevorzugt 95,0 bis 100 Gew.-%, weiter bevorzugt 97,5 bis 100 Gew.-%, besonders bevorzugt 99,0 bis 100,0 Gew.-%, ganz besonders bevorzugt 100 Gew.-% ausmacht, dieser Massenanteil dann während einer auf die Anlaufphase folgenden Reduktionsphase auf 85,0 Gew.-% oder weniger, bevorzugt 0 bis 80,0 Gew.-%, weiter bevorzugt 0,1 bis 50,0 Gew.-%, besonders bevorzugt auf 1,0 bis 40,0 Gew.-%, ganz besonders bevorzugt auf 5,0 bis 30,0 Gew.-% reduziert und dieser Massenanteil dann in einer sich an die Reduktionsphase anschließenden Produktionsphase aufrechterhalten werden kann, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet ist.

In einer bevorzugten Ausführungsform umfasst die Aminstrom-Vorrichtung eine Dosiereinrichtung zur unabhängig voneinander variablen Bereitstellung der Amine hinsichtlich der Massenanteile. Mit anderen Worten umfasst die Aminstrom-Vorrichtung vorzugsweise eine Dosiereinrichtung je Amin (oder Amin-Gemisch, wenn beispielsweise das erste oder zweite Amin bereits selbst ein Amin-Gemisch darstellt), so dass die Massenströme/Massenanteile unabhängig voneinander geregelt werden können. Dies können beispielsweise Dosierpumpen oder Regelventile sein.

Darüber hinaus ist es bevorzugt, dass die Aminstrom-Vorrichtung
i. mindestens jeweils einen Vorlagebehälter für das erste Amin und das zweite Amin,
ii. mindestens jeweils eine den Vorlagebehältern nachgelagerte Dosiereinrichtung,
iii. gegebenenfalls mindestens eine den Dosiereinrichtungen nachgelagerte Mischeinrichtung,
iv. gegebenenfalls mindestens einen Verdampfer zur Verdampfung des Aminstroms,
v. gegebenenfalls einen Wärmetauscher zur Erwärmung des Aminstroms,
umfasst. Die optionale Mischeinrichtung kann dabei beispielsweise ein T-Stück in der Rohrleitung, eine Mischkammer, ein Statikmischer oder eine Pumpe, insbesondere Mischpumpe, oder Rührbehälter sein. Eine Vermischung der Aminströme aus den Dosiereinrichtungen kann jedoch auch im Verdampfer erfolgen. Ein Verdampfer kommt insbesondere bei flüssigen Aminen zum Einsatz.

Die optionalen weiteren organischen Amine, welche von dem ersten und zweiten Amin verschieden sind, können entweder über einen separaten Vorlagebehälter bereitgestellt werden oder mit dem ersten und/oder zweiten Amin gemischt sein. Insbesondere werden hierdurch auch Verunreinigungen, welche in dem ersten und/oder zweiten Amin vorhanden seien können, umfasst.

Vorzugsweise umfasst die Aminstrom-Vorrichtung in wenigstens einer der aminführenden Rohrleitungen wenigstens eine Flanschverbindung, die zur Aufnahme einer Steckscheibe vorbereitet ist. Auf diese Weise wird es ermöglicht, den Aminstrom durch die entsprechende Leitung durch die Steckscheibe sicher zu unterbinden oder die entsprechende Amin-Leitung sicher zu trennen.

Die Phosgenstrom-Vorrichtung, die optionale Inertstoffstrom-Vorrichtung sowie die Aminstrom-Vorrichtung sind jeweils über mindestens eine Zuleitung mit dem Phosgenierungsreaktor verbunden. Die Verbindung der Zuleitungen mit dem Phosgenierungsreaktor kann dabei beispielsweise über einfache Rohrleitungen, Flanschverbindungen, Glattstrahldüsen, Ringspaltdüsen oder ein Mischelement erfolgen. Die Ringspaltdüse kann dabei mit zwei oder mehr, vorzugsweise zwei koaxial angeordneten Kanälen für den Aminstrom und den Phosgenstrom oder einen Inertgasstrom ausgestattet sein, wobei der Aminstrom und der Phosgenstrom erst im Phosgenierungsreaktor aufeinander treffen. Alternativ, insbesondere in der großtechnischen Anwendung, wird das Amin durch ein Einleitrohr, an dessen Ende sich eine zentrale Düse befindet und welches durch einen Deckel sowie gegebenenfalls Halter/Gleichrichter in Position gehalten wird, dem Phosgenierungsreaktor zugegeben so dass sich um das Einleitrohr im Reaktor ein Ringraum bildet, wobei das Phosgen über ein separaten Einlass in den Ringraum und von dort über den Gleichrichter in den Phosgenierungsreaktor gelangt (siehe hierzu auch EP1362847 A2). Der Phosgenierungsreaktor hat eine Mischzone, wo die Ströme erstmals aufeinandertreffen, sowie eine Reaktionszone, wo die Ströme weiter umgesetzt werden. Die Mischzone und Reaktionszone liegen bevorzugt in einem Apparat vor. Ferner ist es bevorzugt, dass der Phosgenierungsreaktor im Wesentlichen senkrecht aufgestellt ist und dieser bevorzugt am oberen Ende mit der Phosgenstrom-Vorrichtung und der Aminstrom-Vorrichtung verbunden ist.

Außerdem umfasst die Phosgenierungsanlage vorzugsweise mindestens eine Quenche zur Abkühlung und zumindest teilweisen Kondensation des Reaktionsgemischs, welche mit dem Phosgenierungsreaktor, vorzugsweise am unteren Ende, verbunden ist, wobei die Quenche insbesondere zur Einbringung von Chlorbenzol, o-Dichlorbenzol und/oder mindestens eines der in der Phosgenierungsanlage hergestellten Isocyanate ausgebildet ist. Insbesondere ist der Quenche ein Auffangbehälter zugeordnet, wobei der Auffangbehälter bevorzugt in der Quenche eingebunden oder separat mit dieser verbunden ist.

### Inbetriebnahme der Phosgenierungsanlage:

Im Rahmen der vorliegenden Erfindung umfasst der Ausdruck "Inbetriebnahme der Phosgeniervorrichtung" alle Verfahrensschritte, die erforderlich sind, um eine außer Betrieb befindliche Phosgeniervorrichtung, zum Beispiel nach einem wartungsbedingten Stillstand, in einen kontinuierlichen Regelbetrieb zur Phosgenierung des entsprechenden Amins bzw. der entsprechenden Amine zu überführen.

Im Rahmen der Erfindung werden zur Inbetriebnahme (Anfahren) der Phosgenierungsanlage zumindest die folgenden Schritte durchlaufen:
(A) Beschickung des Phosgenierungsreaktors mit dem gasförmigen Phosgenstrom und gegebenenfalls des Inertstoffstroms sowie anschließend kontinuierliche Zufuhr des gasförmigen Phosgenstroms und gegebenenfalls des Inertstoffstroms in den Phosgenierungsreaktor,
(B) Kontinuierliche Zufuhr des Aminstroms in den Phosgenierungsreaktor.

Schritt (B) umfasst dabei eine Anlaufphase, eine sich hieran anschließende Reduktionsphase und eine der Reduktionsphase nachgelagerte Produktionsphase, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird.

In einer bevorzugten Ausführungsform erfolgt vor Schritt (A) eine Vorwärmphase, während der ein erhitztes Inertgas, vorzugsweise Stickstoff, durch die Aminstromzuleitung und/oder die Phosgenstromzuleitung in den Reaktor geleitet wird.

In einer bevorzugten Ausführungsform macht der Massenanteil des ersten Amins an der Gesamtheit der Amine im Aminstrom während der Anlaufphase 95,0 bis 100,0 Gew.-%, bevorzugt 97,5 bis 100,0 Gew.-%, besonders bevorzugt 99,0 bis 100,0 Gew.-%, ganz besonders bevorzugt 100 Gew.-% aus. In einigen Fällen, beispielsweise wenn das erste Amin und das zweite Amin nicht über eine separate Zuleitung zum Reaktor verfügt, kann es einen unverhältnismäßigen Spülaufwand bedeuten, Reste anderer Amine zu entfernen, bevor die Phosgeniervorrichtung in Betrieb genommen wird. Deshalb beträgt in solchen Fällen der Startwert für den Massenanteil des ersten Amins am Gesamtstrom der Amine, der der Phosgeniervorrichtung zugeführt wird, maximal 99,9%, bevorzugt maximal 99,7% und besonders bevorzugt maximal 99,5%. Vorzugsweise beträgt die Dauer der Anlaufphase mindestens 5 Minuten beträgt, bevorzugt 10 bis 2880 Minuten, weiter bevorzugt 15 bis 1440 Minuten, besonders bevorzugt 20 bis 240, am meisten bevorzugt 30 bis 120 Minuten.

Während der Reduktionsphase ist es bevorzugt, dass der Massenanteil des ersten Amins an der Gesamtheit der Amine im Aminstrom auf 0 bis 80,0 Gew.-% reduziert wird, weiter bevorzugt auf 0,1 bis 50,0 Gew.-%, besonders bevorzugt auf 1,0 bis 40,0 Gew.-%, ganz besonders bevorzugt auf 5,0 bis 30,0 Gew.-%. Es ist weiterhin bevorzugt, dass die Reduzierung des Massenanteils von dem ersten Amin in der Reduktionsphase schrittweise oder kontinuierlich erfolgt. Während der Reduktionsphase kann der Massenanteil des ersten Amins zweitweise auch unter den Wert reduziert werden, der in der Produktionsphase aufrechterhalten wird. Vorzugsweise beträgt die Dauer der Reduktionsphase mindestens 0,01 Minuten, weiter bevorzugt mindestens 0,1 bis 240 Minuten, besonders bevorzugt 0,5 bis 60 Minuten, ganz besonders bevorzugt 1 bis 30 Minuten.

Der Phosgenstrom und gegebenenfalls der Interstoffstrom weist bei der Beschickung und kontinuierlichen Zufuhr in Schritt (A) vorzugsweise eine Temperatur von 200 °C bis 600 °C, weiter bevorzugt von 250 °C bis 500 °C, besonders bevorzugt von 250 °C bis 330 °C auf. Der Schritt A) kann auch als Aufheizphase bezeichnet werden. In einer bevorzugten Ausführungsform liegt das Phosgen in dem Phosgenierungsreaktor in einem molaren Überschuss in Bezug auf die Aminogruppen der Amine von 30 bis 330 %, bevorzugt 60 bis 300 % und besonders bevorzugt 90 bis 270 % vor.

Der Aminstrom weist bei der bei der Zufuhr in den Phosgenierungsreaktor in Schritt (B) bevorzugt eine Temperatur von 200 °C bis 600 °C, weiter bevorzugt von 250 °C bis 500 °C, ganz besonders bevorzugt von 250 °C bis 330 °C auf. In einer bevorzugten Ausführungsform wird mit der Zufuhr des Aminstroms erst begonnen, wenn der Phosgenierungsreaktor mit Phosgen und gegebenenfalls Inertstoff beschickt ist und die gewünschte Starttemperatur von 230 °C bis 320 °C, vorzugsweise 240 bis 310 °C erreicht wurde.

Der Inertstoff ist vorzugsweise ausgewählt aus der Gruppe, umfassend oder bestehend aus Inertgasen, inerten Lösungsmitteln oder Mischungen von diesen. Das Inertgas kann dabei ausgewählt sein aus der Gruppe, umfassend oder bestehend aus Stickstoff, Argon oder Mischungen von diesen. Das Lösungsmittel ist vorzugsweise ausgewählt aus der Gruppe, umfassend oder bestehend aus aromatischen Kohlenwasserstoffen, bevorzugt Chlorbenzol, o-Dichlorbenzol oder Mischungen von diesen.

Sofern mehr als ein Amin zur Reaktion gebracht wird, ist es möglich, die Amine zunächst zu vermischen und dann die Amin-Mischung zu verdampfen und zu erhitzen. Alternativ kann eines oder mehrere Amine separat verdampft und erhitzt werden. Auch die Einspeisung in den Phosgenierungsreaktor kann für verschiedene Amine separat durch verschiedene Einspeisevorrichtungen (Zuleitungen) erfolgen oder durch das Einspeisen des gemischten Aminstroms. Auch bei separater Einspeisung von zwei oder mehr Aminen in den Phosgenierungsreaktor wird die Summe dieser Aminströme vorliegend als Gesamtstrom von Aminen betrachtet, der dem Phosgenierungsreaktor zugeführt wird, so dass sich Anteile des ersten Amins am Gesamtstrom der Amine jeweils eben auf diesen Gesamtstrom der Amine, der dem Reaktor zu einer bestimmten Zeit zugeführt wird, bezieht, unabhängig davon, ob dies als Mischung oder in Form einer separaten Zuführung/Zuleitung erfolgt. Vorzugsweise werden die Amine aus separaten Vorlagebehältern zunächst vermischt und dann gemeinsam verdampft, erhitzt und in den Phosgenierungsreaktor eingespeist.

### Amine:

Das erste Amin ist ausgewählt aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon

Das zweite Amin ist ausgewählt aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, 1-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon.

Unter den optionalen weiteren organischen Aminen, welche von dem ersten und zweiten Amin verschieden sind, werden im Rahmen der Erfindungen primäre, sekundäre und tertiäre aliphatische, aromatische und heterocyclische Amine verstanden. Zum Beispiel sind weitere organische Amine 1-Aminohexan, 1,2-Diaminocyclohexan, 1,4-Diaminocyclohexan, 6-Aminocapronitril, Bis(hexamethylen)triamin, Azepan, Azepin, 2-Aminomethylcyclopentylamin, 2-Methylpentamethylendiamin, 2,3,4,5-Tetrahydropyridin, Piperidin, Tri-n-butylamin oder Mischungen davon.

### Ausführungsformen:

Die vorliegende Erfindung betrifft insbesondere die folgenden Ausführungsformen:
Nach einer ersten Ausführungsform betrifft die Erfindung ein Verfahren zur Herstellung von Isocyanaten durch Gasphasenphosgenierung der korrespondierenden Amine in einer Phosgenierungsanlage, welche
(a) mindestens eine Phosgenstrom-Vorrichtung 20 zur Bereitstellung eines gasförmigen Phosgenstroms, gegebenenfalls umfassend neben Phosgen einen Inertstoff im Phosgenstrom
(b) gegebenenfalls eine Inertstoffstrom-Vorrichtung zur Bereitstellung eines Inertstoffstroms,
(c) mindestens eine Aminstrom-Vorrichtung 10 zur Bereitstellung eines gasförmigen Aminstroms, gegebenenfalls umfassend neben Aminen einen Inertstoff im Aminstrom;
(d) einen Phosgenierungsreaktor 1 zur Vermischung der Ströme aus (a) bis (c) und Umsetzung des Aminstroms mit dem Phosgenstrom,
umfasst oder daraus besteht, wobei zur Inbetriebnahme der Phosgenierungsanlage die folgenden Schritte durchlaufen werden:
(A) Beschickung des Phosgenierungsreaktors 1 mit dem gasförmigen Phosgenstrom und gegebenenfalls des Inertstoffstroms sowie anschließend kontinuierliche Zufuhr des gasförmigen Phosgenstroms und gegebenenfalls des Inertstoffstroms in den Phosgenierungsreaktor 1,
(B) Kontinuierliche Zufuhr des Aminstroms in den Phosgenierungsreaktor 1,
dadurch gekennzeichnet, dass der Schritt (B) eine Anlaufphase, eine sich hieran anschließende Reduktionsphase und eine der Reduktionsphase nachgelagerte Produktionsphase umfasst, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor 1 zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird und wobei
- das erste Amin ausgewählt ist aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon; und
- das zweite Amin ausgewählt ist aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, 1-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon.

Nach einer zweiten Ausführungsform betrifft die Erfindung ein Verfahren gemäß Ausführungsform 1, dadurch gekennzeichnet, dass die Dauer der Anlaufphase mindestens 5 Minuten beträgt, bevorzugt 10 bis 2880 Minuten, weiter bevorzugt 15 bis 1440 Minuten, besonders bevorzugt 20 bis 240, am meisten bevorzugt 30 bis 120 Minuten.

Nach einer dritten Ausführungsform betrifft die Erfindung ein Verfahren gemäß Ausführungsform 1 oder 2, dadurch gekennzeichnet, dass die Reduzierung des Massenanteils von dem ersten Amin in der Reduktionsphase schrittweise oder kontinuierlich erfolgt.

Nach einer vierten Ausführungsform betrifft die Erfindung ein Verfahren gemäß einer der vorstehenden Ausführungsformen, dadurch gekennzeichnet, dass die Dauer der Reduktionsphase mindestens 0,01 Minuten beträgt, bevorzugt mindestens 0,1 bis 240 Minuten, weiter bevorzugt 0,5 bis 60 Minuten, besonders bevorzugt 1 bis 30 Minuten.

Nach einer fünften Ausführungsform betrifft die Erfindung ein Verfahren gemäß einer der vorstehenden Ausführungsformen, dadurch gekennzeichnet, dass während der Anlaufphase der Massenanteil des ersten Amins an der Gesamtheit der Amine im Aminstrom 95,0 bis 100,0 Gew.-%, bevorzugt 97,5 bis 100,0 Gew.-%, besonders bevorzugt 99,0 bis 100,0 Gew.-%, ganz besonders bevorzugt 100 Gew.-% ausmacht.

Nach einer sechsten Ausführungsform betrifft die Erfindung ein Verfahren gemäß einer der vorstehenden Ausführungsformen, dadurch gekennzeichnet, dass während der Reduktionsphase der Massenanteil des ersten Amins an der Gesamtheit der Amine im Aminstrom auf 0 bis 80,0 Gew.-% reduziert wird, bevorzugt auf 0,1 bis 50,0 Gew.-%, weiter bevorzugt auf 1,0 bis 40,0 Gew.-%, besonders bevorzugt auf 5,0 bis 30,0 Gew.-%.

Nach einer siebten Ausführungsform betrifft die Erfindung ein Verfahren gemäß einer der vorstehenden Ausführungsformen, dadurch gekennzeichnet, dass der Phosgenstrom und gegebenenfalls der Inertstoffstrom bei der Beschickung und kontinuierlichen Zufuhr in Schritt (A) eine Temperatur von 200 °C bis 600 °C, bevorzugt von 250 °C bis 500 °C, ganz besonders bevorzugt von 250 °C bis 330 °C aufweist.

Nach einer achten Ausführungsform betrifft die Erfindung ein Verfahren gemäß einer der vorstehenden Ausführungsformen, dadurch gekennzeichnet, dass der Aminstrom bei der Zufuhr in den Phosgenierungsreaktor 1 in Schritt (B) eine Temperatur von 200 °C bis 600 °C, bevorzugt von 250 °C bis 500 °C, ganz besonders bevorzugt von 250 °C bis 330 °C aufweist.

Nach einer neunten Ausführungsform betrifft die Erfindung ein Verfahren gemäß einer der vorstehenden Ausführungsformen, dadurch gekennzeichnet, dass das Phosgen in dem Phosgenierungsreaktor 1 in einem molaren Überschuss in Bezug auf die Aminogruppen der Amine von 30 bis 330 %, bevorzugt 60 bis 300 % und besonders bevorzugt 90 bis 270 % vorliegt.

Nach einer zehnten Ausführungsform betrifft die Erfindung ein Verfahren gemäß einer der vorstehenden Ausführungsformen, dadurch gekennzeichnet, dass der Inertstoff ausgewählt ist aus der Gruppe, umfassend oder bestehend aus Inertgasen, inerten Lösungsmitteln oder Mischungen von diesen.

Nach einer elften Ausführungsform betrifft die Erfindung ein Verfahren gemäß Ausführungsform 10, dadurch gekennzeichnet, dass der Inertstoff ausgewählt ist aus der Gruppe, umfassend oder bestehend aus Inertgasen, inerten Lösungsmitteln oder Mischungen von diesen.

Nach einer zwölften Ausführungsform betrifft die Erfindung ein Verfahren gemäß Ausführungsform 10 oder 11, dadurch gekennzeichnet, dass das Lösungsmittel ausgewählt ist aus der Gruppe, umfassend oder bestehend aus aromatischen Kohlenwasserstoffen, bevorzugt Chlorbenzol, o-Dichlorbenzol oder Mischungen von diesen.

Nach einer dreizehnten Ausführungsform betrifft die Erfindung eine Phosgenierungsanlage zur Durchführung des Verfahrens gemäß den Ausführungsformen 1 bis 12, umfassend oder bestehend aus
(a) mindestens eine Phosgenstrom-Vorrichtung 20 zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung 10 zur Bereitstellung eines gasförmigen Aminstroms, gegebenenfalls umfassend neben Aminen einen Inertstoff im Aminstrom; und
(d) einen Phosgenierungsreaktor 1 zur Vermischung der Ströme aus (a) bis (c) und Umsetzung des Aminstroms mit dem Phosgenstrom;
dadurch gekennzeichnet, dass die Aminstrom-Vorrichtung 10 ausgestaltet ist, den Aminstrom in der Weise bereitzustellen, dass in einer Anlaufphase der Aminstrom mindestens ein erstes Amin sowie optional ein zweites Amin umfasst und der Massenanteil des ersten Amins an der Gesamtheit der Amine im Aminstrom, welcher dem Phosgenierungsreaktor 1 zugeführt werden kann, mindestens 90,0 Gew.-%, bevorzugt 95,0 bis 100 Gew.-%, weiter bevorzugt 97,5 bis 100 Gew.-%, besonders bevorzugt 99,0 bis 100,0 Gew.-%, ganz besonders bevorzugt 100 Gew.-% ausmacht, dieser Massenanteil dann während einer auf die Anlaufphase folgenden Reduktionsphase auf 85,0 Gew.-% oder weniger, bevorzugt 0 bis 80,0 Gew.-%, weiter bevorzugt 0,1 bis 50,0 Gew.-%, besonders bevorzugt auf 1,0 bis 40,0 Gew.-%, ganz besonders bevorzugt auf 5,0 bis 30,0 Gew.-% reduziert und dieser Massenanteil dann in einer sich an die Reduktionsphase anschließenden Produktionsphase aufrechterhalten werden kann, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet ist.

Nach einer vierzehnten Ausführungsform betrifft die Erfindung eine Vorrichtung gemäß Ausführungsform 13, dadurch gekennzeichnet, dass die Phosgenstrom-Vorrichtung 20 mindestens einen Wärmetauscher 21 zur Erwärmung des Phosgenstroms und/oder des Inertstoffstroms umfasst.

Nach einer fünfzehnten Ausführungsform betrifft die Erfindung eine Vorrichtung gemäß Ausführungsform 13 oder 14, dadurch gekennzeichnet, dass die Aminstrom-Vorrichtung 10 eine Dosiereinrichtung 13 zur unabhängig voneinander variablen Bereitstellung der Amine hinsichtlich der Massenanteile umfasst.

Nach einer sechszehnten Ausführungsform betrifft die Erfindung eine Vorrichtung gemäß einer der Ausführungsformen 13 bis 15, dadurch gekennzeichnet, dass die Aminstrom- Vorrichtung 10
i. mindestens jeweils einen Vorlagebehälter 11, 12 für das erste Amin und das zweite Amin,
ii. mindestens jeweils eine den Vorlagebehältern 11, 12 nachgelagerte Dosiereinrichtung 13,
iii. gegebenenfalls mindestens eine den Dosiereinrichtungen 13 nachgelagerte Mischeinrichtung 14,
iv. gegebenenfalls mindestens einen Verdampfer 15 zur Verdampfung des Aminstroms,
v. gegebenenfalls einen Wärmetauscher 16 zur Erwärmung des Aminstroms, umfasst.

Nach einer siebzehnten Ausführungsform betrifft die Erfindung eine Vorrichtung gemäß einer der Ausführungsformen 13 bis 16, dadurch gekennzeichnet, dass der Phosgenierungsreaktor 1 im Wesentlichen senkrecht aufgestellt ist und dieser bevorzugt am oberen Ende mit der Phosgenstrom-Vorrichtung 20 und der Aminstrom-Vorrichtung 10 verbunden ist.

Nach einer achtzehnten Ausführungsform betrifft die Erfindung eine Vorrichtung gemäß einer der Ausführungsformen 13 bis 17, dadurch gekennzeichnet, dass die Phosgenierungsanlage mindestens eine Quenche 2 zur Abkühlung und zumindest teilweisen Kondensation des Reaktionsgemischs umfasst, welche mit dem Phosgenierungsreaktor 1, vorzugsweise am unteren Ende, verbunden ist, wobei die Quenche 2 insbesondere zur Einbringung von Chlorbenzol, o-Dichlorbenzol und/oder mindestens eines der in der Phosgenierungsanlage hergestellten Isocyanate ausgebildet ist.

Nach einer neunzehnten Ausführungsform betrifft die Erfindung eine Vorrichtung gemäß Ausführungsform 18, dadurch gekennzeichnet, dass der Quenche 2 ein Auffangbehälter zugeordnet ist, wobei der Auffangbehälter insbesondere in der Quenche eingebunden oder separat mit dieser verbunden ist.

Nach einer zwanzigsten Ausführungsform betrifft die Erfindung ein Verfahren zum Anfahren einer Phosgenierungsanlage, welche
(a) mindestens eine Phosgenstrom-Vorrichtung 20 zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung 10 zur Bereitstellung eines gasförmigen Aminstroms, gegebenenfalls umfassend neben Aminen einen Inertstoff im Aminstrom; und
(d) einen Phosgenierungsreaktor 1 zur Vermischung der Ströme aus (a) bis (c) und Umsetzung des Aminstroms mit dem Phosgenstrom;
umfasst oder daraus besteht, wobei zur Inbetriebnahme der Phosgenierungsanlage die folgenden Schritte durchlaufen werden:
(A) Beschickung des Phosgenierungsreaktors 1 mit dem gasförmigen Phosgenstrom und gegebenenfalls des Inertstoffstroms sowie anschließend kontinuierliche Zufuhr des gasförmigen Phosgenstroms und gegebenenfalls des Inertstoffstroms in den Phosgenierungsreaktor 1,
(B) Kontinuierliche Zufuhr des Aminstroms in den Phosgenierungsreaktor 1,
dadurch gekennzeichnet, dass der Schritt (B) eine Anlaufphase, eine sich hieran anschließende Reduktionsphase und eine der Reduktionsphase nachgelagerte Produktionsphase umfasst, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor 1 zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird und
- das erste Amin ausgewählt ist aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon; und
- das zweite Amin ausgewählt ist aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, l-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon..

### Beispiele:

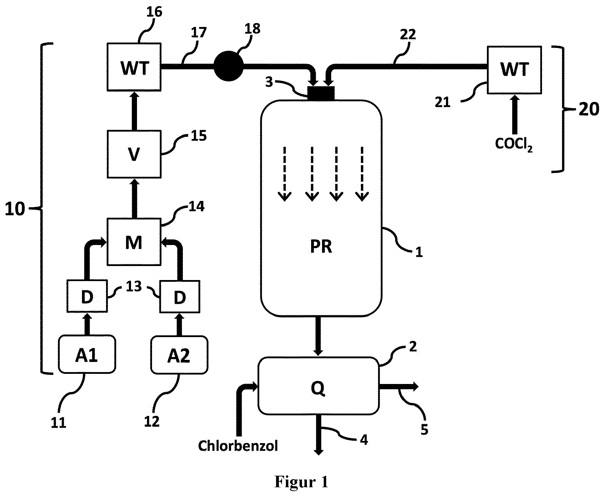

Die vorliegende Erfindung wird im Folgenden anhand der Figur 1 sowie der nachfolgenden Beispielen erörtert, ist jedoch nicht auf diese beschränkt. Es zeigt
- Figur 1:: eine schematische Darstellung einer erfindungsgemäßen Phosgenierungsanlage.

Figur 1 zeigt eine schematische Darstellung einer erfindungsgemäßen Phosgenierungsanlage. Diese umfasst einen senkrecht aufgestellten Rohrreaktor/Phosgenierungsreaktor (PR) 1 mit einer Verweilzeitstrecke zur Umsetzung eines Aminstroms mit einem Phosgenstrom und einer daran anschließenden Quenche (Q) mit Auffangbehälter 2 zur Abkühlung und zumindest teilweisen Kondensation der in dem Phosgenierungsreaktor 1 entstehenden Reaktionsgase, eine Aminstrom-Vorrichtung 10 zur Bereitstellung des Aminstroms sowie eine Phosgenstrom-Vorrichtung 20 zur Bereitstellung des Phosgenstroms. Die Quenche 2 verfügt über einen Produktauslass 4 zur Weiterleitung des flüssigen Rohprodukts sowie einen Gasauslass 5 zur Ableitung der Abgase.

Die Aminstrom-Vorrichtung 10 besteht aus einem ersten und zweiten Vorlagebehälter 11, 12 für ein erstes (A1) beziehungsweise ein zweites Amin (A2) sowie jeweils eine den Vorlagebehältern 11, 12 nachgelagerte Dosiereinrichtung (D) 13 zur unabhängig voneinander variablen Einstellung der Mengenverhältnisse (beziehungsweise Massenanteile) der Amine (A1), (A2) aus dem ersten und zweiten Vorlagebehälter 11, 12. Stromabwärts der Dosiereinrichtung 13 ist eine Mischeinrichtung (M) 14 zur Vermischung der Aminströme angeordnet, wobei der Mischeinrichtung 14 ein Verdampfer (V) 15 zur Verdampfung des Aminstroms nachgelagert ist. Dem Verdampfer 15 ist wiederum ein Wärmetauscher (WT) 16 zur Erhitzung des Aminstroms nachgelagert.

Die Phosgenstrom-Vorrichtung 20 umfasst einen Wärmetauscher (WT) 21 zur Erhitzung des Phosgenstroms.

Die Aminstrom-Vorrichtung 10 ist über eine Aminstrom-Zuleitung 17 und die Phosgenstrom-Vorrichtung 20 über eine Phosgenstron-Zuleitung 22 mit dem Phosgenierungsreaktor 1 verbunden, wobei die Zuleitungen in eine im Kopfbereich des Phosgenierungsreaktors 1 vorgesehene Ringspaltdüse 3 mit zwei koaxial angeordneten Kanälen münden. Durch die getrennte Einspeisung des Phosgenstroms und des Aminstroms kann eine Vermischung und Umsetzung dieser Reaktanden unmittelbar nach dem Eindüsen in den Phosgenierungsreaktor erfolgen. Die Aminstrom-Zuleitung 17 der Aminstrom-Vorrichtung 10 ist zudem mit einem Druckmesser 18 zur Messung des Drucks in der Aminstrom-Zuleitung 17 ausgestattet.

### Allgemeine Arbeitsvorschrift:

Die folgenden Beispiele wurden in einer Versuchsanlage zur Gasphasenphosgenierung gemäß Figur 1 durchgeführt. Phosgen wurde in dem Wärmetauscher 21 auf ca. 340 °C erhitzt und mit dieser Temperatur in den Phosgenierungsreaktor 1 über die Phosgenstrom-Zuleitung 22 und der Ringspaltdüse 3 eingeleitet. Amin-Seitig wurde nach Bedarf das erste und/oder das zweite Amin aus den Vorlagebehältern 11 und 12 jeweils unabhängig voneinander über eine Dosiereinrichtung 13 dosiert, so dass die gewünschten Massenanteile erzielt werden. Nach Durchlaufen der Dosiereinrichtung 13 wurden die Amine in einer Mischeinrichtung 14 vermischt, anschließend in einem Verdampfer 15 verdampft, dann in einem Wärmetauscher 16 auf ca. 330 °C überhitzt und mit dieser Temperatur in den Phosgenierungsreaktor 1 über die Aminstrom-Zuleitung 17 und der Ringspaltdüse 3 eingeleitet. Der molare Phosgenüberschuss betrug je nach eingesetztem Amin bzw. eingesetzter Amin-Mischung zwischen 140% und 300%, bezogen auf die Aminogruppen des Amins. Zum Anfahren der kontinuierlichen Reaktion wurde der Phosgenierungsreaktor 1 zunächst nur mit heißem Phosgen beaufschlagt, bis sich eine konstante Temperatur eingestellt hatte. Erst dann wurde mit der Zuführung des Amin-Dampfs begonnen. Die Reaktion erfolgte abgesehen von unvermeidlichen Wärmeverlusten adiabat. Nach Durchlaufen der Verweilzeitstrecke wurden die Reaktionsgase in der Quenche 2 durch Einbringen von Chlorbenzol abgekühlt und zumindest teilweise kondensiert. In der Aminstrom-Zuleitung 17 wurde der Druck über einen Druckmesser 18 beobachtet.

### Vergleichsbeispiel 1

Der Phosgenierungsreaktor 1 wurde wie in der allgemeinen Arbeitsvorschrift beschrieben mittels Phosgendampf auf die Starttemperatur aufgeheizt. Dann wurde begonnen, aus den beiden Amin-Vorlagen eine 20:80 Mischung (Gewichtsverhältnis) von ISODA zu IPDA über die Dosiereinrichtungen 13 zur Mischeinrichtung 14 dosiert und so die Phosgenierungsreaktion gestartet. Im Phosgenierungsreaktor 1 bildeten sich Ablagerungen, was zu einem raschen und sich beschleunigenden Anstieg des Drucks in der Aminstrom-Zuleitung 17 der Aminstrom-Vorrichtung 10 führte. Nach ca. 9 Minuten war der Druck von anfangs ca. 1000 mbar auf 1500 mbar angestiegen und die Reaktion wurde beendet.

### Beispiel 1

Der Phosgenierungsreaktor 1 wurde vorbereitet wie in dem Vergleichsbeispiel 1. Jedoch wurde nach der Aufheizphase in einer Anlaufphase zunächst nur IPDA über die Dosiereinrichtungen 13 zur Mischeinrichtung 14 dosiert, also ISODA zu IPDA von 0:100 (Gewichtsverhältnis), um die Reaktion zunächst mit IPDA zu starten. Nach 60 Minuten kontinuierlicher Betriebszeit wurde die Reduktionsphase eingeleitet. Dabei wurde die Fördermenge an IPDA reduziert und gleichzeitig mit der Dosierung von ISODA begonnen, bis eine 10:90 Mischung (Gewichtsverhältnis) aus ISODA zu IPDA zur Reaktion gebracht wurde. Nach weiteren 30 Minuten wurden die Amin-Fördermengen weiter angepasst, so dass das Gewichtsverhältnis von ISODA zu IPDA nun 20:80 (Gewichtsverhältnis) betrug, also genau das Verhältnis aus Vergleichsbeispiel 1. Es wurde weitere 60 Minuten mit diesem Mischungsverhältnis phosgeniert (Produktionsphase), bevor die Reaktion beendet wurde. Der Druck in der Aminstrom-Zuleitung 17 der Aminstrom-Vorrichtung 10 blieb während der Reaktion nahezu konstant und betrug am Ende ca. 1020 mbar (anfangs ca. 1000 mbar).

### Vergleichsbeispiel 2

Der Phosgenierungsreaktor 1 wurde wie in der allgemeinen Arbeitsvorschrift beschrieben mittels Phosgendampf auf die Starttemperatur aufgeheizt. Dann wurde die Reaktion gestartet, indem PDA verdampft und über die Düse in den Phosgenierungsreaktor 1 gefahren wurde, entsprechend einem Gewichtsverhältnis PDA zu IPDA von 100:0. Es wurde ein rascher Anstieg des Drucks in der Aminstrom-Zuleitung 17 der Aminstrom-Vorrichtung 10 beobachtet, wobei schon nach ca. 6 min der Grenzwert von 1500 mbar (anfangs ca. 1000 mbar) erreicht wurde, so dass die Reaktion beendet wurde.

### Beispiel 2

Der Phosgenierungsreaktor 1 wurde vorbereitet wie in dem Vergleichsbeispiel 2. Jedoch wurde nach der Aufheizphase zunächst nur IPDA über die Dosiereinrichtungen 13 zur Mischeinrichtung 14 dosiert, also PDA zu IPDA von 0:100 (Gewichtsverhältnis), um die Reaktion zunächst mit IPDA zu starten (Anlaufphase). Nach 1,5 h wurde begonnen, das Gewichtsverhältnis von PDA zu IPDA in 5 gleichen Schritten (20:80, 40:60, 60:40, 80:20 und 100:0) alle 30 Minuten auf den Zielwert von 100: 0 (Gewichtsverhältnis PDA zu IPDA) zu ändern (Reduktionsphase). Die Reaktion wurde noch weitere 1,5 Stunden mit PDA weitergeführt (Produktionsphase), bevor der Versuch bei einem Druck von 1090 mbar in der Aminstrom-Zuleitung 17 der Aminstrom-Vorrichtung 10 beendet wurde. Die Bildung von Ablagerungen war also gegenüber dem Vergleichsbeispiel 2 erheblich verlangsamt.

### Bezugszeichenliste

- **1**: Phosgenierungsreaktor/Rohrreaktor (PR)
- **2**: Quenche mit Auffangbehälter (Q)
- **3**: Ringspaltdüse mit zwei koaxial angeordneten Kanälen
- **4**: Produktauslass
- **5**: Gasauslass
- **10**: Aminstrom-Vorrichtung
- **11**: Vorlagebehälter des ersten Amins (A1)
- **12**: Vorlagebehälter des zweiten Amins (A2)
- **13**: Dosiereinrichtung(en)
- **14**: Mischeinrichtung (M)
- **15**: Verdampfer (V) der Aminstrom-Vorrichtung
- **16**: Wärmetauscher (WT) der Aminstrom-Vorrichtung
- **17**: Aminstrom-Zuleitung
- **18**: Druckmesser
- **20**: Phosgenstrom-Vorrichtung
- **21**: Wärmetauscher (WT) der Phosgenstrom-Vorrichtung
- **22**: Phosgenstrom-Zuleitung

## Patentansprüche

1. Verfahren zur Herstellung von Isocyanaten durch Gasphasenphosgenierung der korrespondierenden Amine in einer Phosgenierungsanlage, welche
(a) mindestens eine Phosgenstrom-Vorrichtung (20) zur Bereitstellung eines gasförmigen Phosgenstroms, gegebenenfalls umfassend neben Phosgen einen Inertstoff im Phosgenstrom
(b) gegebenenfalls eine Inertstoffstrom-Vorrichtung zur Bereitstellung eines Inertstoffstroms,
(c) mindestens eine Aminstrom-Vorrichtung (10) zur Bereitstellung eines gasförmigen Aminstroms, gegebenenfalls umfassend neben Aminen einen Inertstoff im Aminstrom;
(d) einen Phosgenierungsreaktor (1) zur Vermischung der Ströme aus (a) bis (c) und Umsetzung des Aminstroms mit dem Phosgenstrom,
umfasst oder daraus besteht, wobei zur Inbetriebnahme der Phosgenierungsanlage die folgenden Schritte durchlaufen werden:
(A)Beschickung des Phosgenierungsreaktors (1) mit dem gasförmigen Phosgenstrom und gegebenenfalls des Inertstoffstroms sowie anschließend kontinuierliche Zufuhr des gasförmigen Phosgenstroms und gegebenenfalls des Inertstoffstroms in den Phosgenierungsreaktor (1),
(B) Kontinuierliche Zufuhr des Aminstroms in den Phosgenierungsreaktor (1),
**dadurch gekennzeichnet, dass** der Schritt (B) eine Anlaufphase, eine sich hieran anschließende Reduktionsphase und eine der Reduktionsphase nachgelagerte Produktionsphase umfasst, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor (1) zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird und wobei
- das erste Amin ausgewählt ist aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon; und
- das zweite Amin ausgewählt ist aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, 1-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon.

2. Verfahren gemäß Anspruch 1, **dadurch gekennzeichnet, dass** die Dauer der Anlaufphase mindestens 5 Minuten beträgt, bevorzugt 10 bis 2880 Minuten, weiter bevorzugt 15 bis 1440 Minuten, besonders bevorzugt 20 bis 240, am meisten bevorzugt 30 bis 120 Minuten und/oder, dass die Dauer der Reduktionsphase mindestens 0,01 Minuten beträgt, bevorzugt mindestens 0,1 bis 240 Minuten, weiter bevorzugt 0,5 bis 60 Minuten, besonders bevorzugt 1 bis 30 Minuten.

3. Verfahren gemäß Anspruch 1 oder 2, **dadurch gekennzeichnet, dass** während der Anlaufphase der Massenanteil des ersten Amins an der Gesamtheit der Amine im Aminstrom 95,0 bis 100,0 Gew.-%, bevorzugt 97,5 bis 100,0 Gew.-%, besonders bevorzugt 99,0 bis 100,0 Gew.-%, ganz besonders bevorzugt 100 Gew.-% ausmacht.

4. Verfahren gemäß einem der vorstehenden Ansprüche, **dadurch gekennzeichnet, dass** während der Reduktionsphase der Massenanteil des ersten Amins an der Gesamtheit der Amine im Aminstrom auf 0 bis 80,0 Gew.-% reduziert wird, bevorzugt auf 0,1 bis 50,0 Gew.-%, weiter bevorzugt auf 1,0 bis 40,0 Gew.-%, besonders bevorzugt auf 5,0 bis 30,0 Gew.-%.

5. Verfahren gemäß einem der vorstehenden Ansprüche, **dadurch gekennzeichnet, dass** der Phosgenstrom und gegebenenfalls der Inertstoffstrom bei der Beschickung und kontinuierlichen Zufuhr in Schritt (A) eine Temperatur von 200 °C bis 600 °C, bevorzugt von 250 °C bis 500 °C, ganz besonders bevorzugt von 250 °C bis 330 °C aufweist und/oder dass der Aminstrom bei der Zufuhr in den Phosgenierungsreaktor (1) in Schritt (B) eine Temperatur von 200 °C bis 600 °C, bevorzugt von 250 °C bis 500 °C, ganz besonders bevorzugt von 250 °C bis 330 °C aufweist.

6. Verfahren gemäß einem der vorstehenden Ansprüche, **dadurch gekennzeichnet, dass** das Phosgen in dem Phosgenierungsreaktor (1) in einem molaren Überschuss in Bezug auf die Aminogruppen der Amine von 30 bis 330 %, bevorzugt 60 bis 300 % und besonders bevorzugt 90 bis 270 % vorliegt.

7. Verfahren gemäß einem der vorstehenden Ansprüche, **dadurch gekennzeichnet, dass** der Inertstoff ausgewählt ist aus der Gruppe, umfassend oder bestehend aus Inertgasen, inerten Lösungsmitteln oder Mischungen von diesen.

8. Verfahren gemäß Anspruch 7, **dadurch gekennzeichnet, dass** das Inertgas ausgewählt ist aus der Gruppe, umfassend oder bestehend aus Stickstoff, Argon oder Mischungen von diesen und/oder, dass das Lösungsmittel ausgewählt ist aus der Gruppe, umfassend oder bestehend aus aromatischen Kohlenwasserstoffen, bevorzugt Chlorbenzol, o-Dichlorbenzol oder Mischungen von diesen.

9. Phosgenierungsanlage zur Durchführung des Verfahrens gemäß den Ansprüchen 1 bis 8, umfassend oder bestehend aus
(a) mindestens eine Phosgenstrom-Vorrichtung (20) zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung (10) zur Bereitstellung eines gasförmigen Aminstroms, gegebenenfalls umfassend neben Aminen einen Inertstoff im Aminstrom; und
(d) einen Phosgenierungsreaktor (1) zur Vermischung der Ströme aus (a) bis (c) und Umsetzung des Aminstroms mit dem Phosgenstrom;
**dadurch gekennzeichnet, dass** die Aminstrom-Vorrichtung (10) ausgestaltet ist, den Aminstrom in der Weise bereitzustellen, dass in einer Anlaufphase der Aminstrom mindestens ein erstes Amin sowie optional ein zweites Amin umfasst und der Massenanteil des ersten Amins an der Gesamtheit der Amine im Aminstrom, welcher dem Phosgenierungsreaktor (1) zugeführt werden kann, mindestens 90,0 Gew.-%, bevorzugt 95,0 bis 100 Gew.-%, weiter bevorzugt 97,5 bis 100 Gew.-%, besonders bevorzugt 99,0 bis 100,0 Gew.-%, ganz besonders bevorzugt 100 Gew.-% ausmacht, dieser Massenanteil dann während einer auf die Anlaufphase folgenden Reduktionsphase auf 85,0 Gew.-% oder weniger, bevorzugt 0 bis 80,0 Gew.-%, weiter bevorzugt 0,1 bis 50,0 Gew.-%, besonders bevorzugt auf 1,0 bis 40,0 Gew.-%, ganz besonders bevorzugt auf 5,0 bis 30,0 Gew.-% reduziert und dieser Massenanteil dann in einer sich an die Reduktionsphase anschließenden Produktionsphase aufrechterhalten werden kann, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet ist.

10. Vorrichtung gemäß Anspruch 9, **dadurch gekennzeichnet, dass** die Phosgenstrom-Vorrichtung mindestens einen Wärmetauscher (21) zur Erwärmung des Phosgenstroms und/oder des Inertstoffstroms umfasst.

11. Vorrichtung gemäß Anspruch 9 oder 10, **dadurch gekennzeichnet, dass** die Aminstrom-Vorrichtung eine Dosiereinrichtung (13) zur unabhängig voneinander variablen Bereitstellung der Amine hinsichtlich der Massenanteile umfasst.

12. Vorrichtung gemäß einem der Ansprüche 9 bis 11, **dadurch gekennzeichnet, dass** die Aminstrom- Vorrichtung (10)
i. mindestens jeweils einen Vorlagebehälter (11, 12) für das erste Amin und das zweite Amin,
ii. mindestens jeweils eine den Vorlagebehältern (11, 12) nachgelagerte Dosiereinrichtung (13),
iii. gegebenenfalls mindestens eine den Dosiereinrichtungen (13) nachgelagerte Mischeinrichtung (14),
iv. gegebenenfalls mindestens einen Verdampfer (15) zur Verdampfung des Aminstroms,
v. gegebenenfalls einen Wärmetauscher (16) zur Erwärmung des Aminstroms,
umfasst.

13. Vorrichtung gemäß einem der Ansprüche 9 bis 12, **dadurch gekennzeichnet, dass** die Phosgenierungsanlage mindestens eine Quenche (2) zur Abkühlung und zumindest teilweisen Kondensation des Reaktionsgemischs umfasst, welche mit dem Phosgenierungsreaktor (1), vorzugsweise am unteren Ende, verbunden ist, wobei die Quenche (2) insbesondere zur Einbringung von Chlorbenzol, o-Dichlorbenzol und/oder mindestens eines der in der Phosgenierungsanlage hergestellten Isocyanate ausgebildet ist.

14. Vorrichtung gemäß Anspruch 13, **dadurch gekennzeichnet, dass** der Quenche (2) ein Auffangbehälter zugeordnet ist, wobei der Auffangbehälter insbesondere in der Quenche (2) eingebunden oder separat mit dieser verbunden ist.

15. Verfahren zum Anfahren einer Phosgenierungsanlage, welche
(a) mindestens eine Phosgenstrom-Vorrichtung (20) zur Bereitstellung eines gasförmigen Phosgenstroms, gegebenenfalls umfassend neben Phosgen einen Inertstoff im Phosgenstrom;
(b) gegebenenfalls eine Inertstoffstrom-Vorrichtung zur Bereitstellung eines Inertstoffstroms;
(c) mindestens eine Aminstrom-Vorrichtung (10) zur Bereitstellung eines gasförmigen Aminstroms, gegebenenfalls umfassend neben Aminen einen Inertstoff im Aminstrom; und
(d) einen Phosgenierungsreaktor (1) zur Vermischung der Ströme aus (a) bis (c) und Umsetzung des Aminstroms mit dem Phosgenstrom;
umfasst oder daraus besteht, wobei zur Inbetriebnahme der Phosgenierungsanlage die folgenden Schritte durchlaufen werden:
(A)Beschickung des Phosgenierungsreaktors (1) mit dem gasförmigen Phosgenstrom und gegebenenfalls des Inertstoffstroms sowie anschließend kontinuierliche Zufuhr des gasförmigen Phosgenstroms und gegebenenfalls des Inertstoffstroms in den Phosgenierungsreaktor (1),
(B) Kontinuierliche Zufuhr des Aminstroms in den Phosgenierungsreaktor (1),
**dadurch gekennzeichnet, dass** der Schritt (B) eine Anlaufphase, eine sich hieran anschließende Reduktionsphase und eine der Reduktionsphase nachgelagerte Produktionsphase umfasst, wobei während der Anlaufphase der Aminstrom mindestens ein erstes Amin sowie optional ein von dem ersten Amin verschiedenes zweites Amin umfasst und der Massenanteil des ersten Amins an der Gesamtheit der Amine im Aminstrom, welcher dem Phosgenierungsreaktor (1) zugeführt wird, mindestens 90,0 Gew.-% ausmacht, dieser Massenanteil dann während der Reduktionsphase auf 85,0 Gew.-% oder weniger reduziert und dieser Massenanteil dann in der Produktionsphase aufrechterhalten wird, wobei während der Anlaufphase, der Reduktionsphase und der Produktionsphase der zu 100 Gew.-% fehlende Rest der Gesamtheit der Amine im Aminstrom von dem zweiten Amin sowie optional zu maximal 1 Gew.-% von weiteren organischen Aminen, welche von dem ersten und zweiten Amin verschieden sind, gebildet wird und
- das erste Amin ausgewählt ist aus der Gruppe, umfassend oder bestehend aus 1,6-Diaminohexan (HDA), 1-Amino-3,5,5-trimethyl-5-aminomethylcyclohexan (IPDA), Bis(p-aminocyclohexyl)methan (PACM) oder Mischungen davon; und
- das zweite Amin ausgewählt ist aus der Gruppe, umfassend oder bestehend aus 1,5-Diaminopentan (PDA), 1,3-Bis(aminomethyl)benzol (m-XDA), 1,4-Bis(aminomethyl)benzol (p-XDA), 1,2-Bis(aminomethyl)cyclohexan (1,2-BAC), 1,3-Bis(aminomethyl)cyclohexan (1,3-BAC), 1,4-Bis(aminomethyl)cyclohexan (1,4-BAC), Tetramethylxylylendiamin (TMXDA), 2,5-Bis(aminomethyl)bicyclo[2.2.1]heptan (2,5-NBDA), 2,6-Bis(aminomethyl)bicyclo[2.2.1]heptan (2,6-NBDA), Neopentandiamin, 2,4,4-Trimethylhexamethylendiamin, 2,2,4-Trimethylhexamethylendiamin, 1,3-Diaminocyclohexan, 1,4-Diaminocyclohexan, 1-Methyl-2,6-diaminocyclohexan und/oder 1-Methyl-2,4-diaminocyclohexan (zusammen oder einzeln H6TDA), 2,5-Diamino-2,5-dideoxy-1,4:3,6-dianhydrosorbitol (ISODA), 2,5-Diamino-2,5-dideoxy-1,4:3,6-dianhydromannitol (MANDA) und 2,5-Diamino-2,5-dideoxy-1,4:3,6-dianhydroiditol (IDDA) oder Mischungen davon.
